# EUROPEAN PATENT APPLICATION

(11) **EP 3 608 404 A1**
(43) Date of publication of application: **12.02.2020**
(21) Application number: 18188296.0
(22) Date of filing: 09.08.2018
(51) Int. Cl.: C12N 9/88, C12N 9/04, C12P 17/12

(54) **AN IMPROVED METHOD TO PRODUCE CHEMICAL COMPOUNDS DERIVED FROM OXALOACETATE BY MICROORGANISMS**

(71) Applicant: Technische Universität München, 80333 München (DE)
(72) Inventor: Marin-Sanguino, Alberto, 85748 Garching (DE); Plfüger-Grau, Katharina, 85748 Garching (DE); Hobmeier, Karina, 85748 Garching (DE); Kremling, Andreas, 85748 Garching (DE)
(74) Representative: Hoefer & Partner Patentanwälte mbB

(57) **Abstract**

A method for the production of a chemical compound derived from oxaloacetate using genetically modified microorganisms is disclosed. The chemical compound can for example be ectoine or the ectoine precursor NADA. The genetic modifications increase the compound production by increasing the extracellular accumulation, decreasing the intracellular degradation, and by disrupting the PEP-pyruvate-oxaloacetate node of the tricarboxylic acid (TCA) cycle. The microorganisms obtained by the genetic modifications are disclosed as well, as well as their use in the production method. The microorganisms are preferably gram negative bacteria, such as halophilic bacteria, for example *Halomonas elongata.*

SEQUENCE LISTING

<110> Technische UniversitÃ¤t MÃ¼nchen

<120> AN IMPROVED METHOD TO PRODUCE CHEMICAL COMPOUNDS DERIVED FROM
OXALOACETATE BY MICROORGANISMS

<130> ETU18XX01PEP

<160> 28

<170> BiSSAP 1.3.6

<210> 1
<211> 43
<212> DNA
<213> Artificial Sequence

<220>
<223> construction of plasmid pSEVA212S-pckA::Sm

<400> 1
atccccgggt accgagctcg gatgtctgga agattacttc agc                43

<210> 2
<211> 28
<212> DNA
<213> Artificial Sequence

<220>
<223> construction of plasmid pSEVA212S-pckA::Sm

<400> 2
gtcaaggttc gtcgattcct gggccttg                                 28

<210> 3
<211> 29
<212> DNA
<213> Artificial Sequence

<220>
<223> construction of plasmid pSEVA212S-pckA::Sm

<400> 3
aggaatcgac gaaccttgac cgaacgcag                                29

<210> 4
<211> 32
<212> DNA
<213> Artificial Sequence

<220>
<223> construction of plasmid pSEVA212S-pckA::Sm

<400> 4
ctcggcaggc ttatttgccg actaccttgg tg                            32

<210> 5
<211> 29
<212> DNA
<213> Artificial Sequence

<220>
<223> construction of plasmid pSEVA212S-pckA::Sm

<400> 5
cggcaaataa gcctgccgag ggcgacaac                                          29

<210> 6
<211> 44
<212> DNA
<213> Artificial Sequence

<220>
<223> construction of plasmid pSEVA212S-pckA::Sm

<400> 6
agggataaca gggtaatctg caggtccaga tgatcgcgac aagg                         44

<210> 7
<211> 28
<212> DNA
<213> Artificial Sequence

<220>
<223> construction of plasmid pSEVA212S-Î\224pckA

<400> 7
ctcggcaggc gtcgattcct gggccttg                                           28

<210> 8
<211> 29
<212> DNA
<213> Artificial Sequence

<220>
<223> construction of plasmid pSEVA212S-Î\224pckA

<400> 8
aggaatcgac gcctgccgag ggcgacaac                                          29

<210> 9
<211> 20
<212> DNA
<213> Artificial Sequence

<220>
<223> PCR/sequencing primer to verify mutant

<400> 9
gaagggcaga tcaccatcac                                                    20

<210> 10
<211> 20
<212> DNA
<213> Artificial Sequence

<220>
<223> PCR/sequencing primer to verify mutant

<400> 10
gtcgagcagt tccacggtag                                                    20

<210> 11
<211> 40
<212> DNA
<213> Artificial Sequence

<220>
<223> construction of plasmid pSEVA212S-maeB::Sm

<400> 11
atccccgggt accgagctcg tccgaagggt acgcgtaccc                              40

<210> 12
<211> 33
<212> DNA
<213> Artificial Sequence

<220>
<223> construction of plasmid pSEVA212S-maeB::Sm

<400> 12
gtcaaggttc gctgaggatc cgatctgcga ttc                                     33

<210> 13
<211> 30
<212> DNA
<213> Artificial Sequence

<220>
<223> construction of plasmid pSEVA212S-maeB::Sm

<400> 13
gatcctcagc gaaccttgac cgaacgcagc                                         30

<210> 14
<211> 32
<212> DNA
<213> Artificial Sequence

<220>

<223> construction of plasmid pSEVA212S-maeB::Sm

<400> 14
ttaagcagta ttatttgccg actaccttgg tg                                    32


<210> 15
<211> 34
<212> DNA
<213> Artificial Sequence


<220>
<223> construction of plasmid pSEVA212S-maeB::Sm

<400> 15
cggcaaataa tactgcttaa atgcctgcac gtgc                                  34


<210> 16
<211> 40
<212> DNA
<213> Artificial Sequence


<220>
<223> construction of plasmid pSEVA212S-maeB::Sm

<400> 16
agggataaca gggtaatctg ctgcgcggcg agatacgtca                            40


<210> 17
<211> 33
<212> DNA
<213> Artificial Sequence


<220>
<223> construction of plasmid pSEVA212S-Î\224maeB

<400> 17
ttaagcagta gctgaggatc cgatctgcga ttc                                   33


<210> 18
<211> 33
<212> DNA
<213> Artificial Sequence


<220>
<223> construction of plasmid pSEVA212S-Î\224maeB

<400> 18
gatcctcagc tactgcttaa atgcctgcac gtg                                   33


<210> 19
<211> 20
<212> DNA
<213> Artificial Sequence

<220>
<223> PCR/sequencing primer to verify mutant

<400> 19
aacaccgaca gccagacatt                                                    20


<210> 20
<211> 18
<212> DNA
<213> Artificial Sequence


<220>
<223> PCR/sequencing primer to verify mutant

<400> 20
ttggacagcg ggagttcg                                                      18


<210> 21
<211> 27
<212> DNA
<213> Artificial Sequence


<220>
<223> construction of plasmid pSEVA212S-Î\224maeA

<400> 21
ttttgaattc gcaaccacga gatcatg                                            27


<210> 22
<211> 32
<212> DNA
<213> Artificial Sequence


<220>
<223> construction of plasmid pSEVA212S-Î\224maeA

<400> 22
agacgaaccg ggaaacactg cttgaatgtc tg                                       32


<210> 23
<211> 30
<212> DNA
<213> Artificial Sequence


<220>
<223> construction of plasmid pSEVA212S-Î\224maeA

<400> 23
cagtgtttcc cggttcgtct ctactgtctc                                         30


<210> 24
<211> 28
<212> DNA

<213> Artificial Sequence

<220>
<223> construction of plasmid pSEVA212S-Î\224maeA

<400> 24
aaaaaagctt ctggatgctc tcctgcgt                                          28

<210> 25
<211> 18
<212> DNA
<213> Artificial Sequence

<220>
<223> PCR/sequencing primer to verify mutant

<400> 25
caagcgggtc cagcagtc                                                     18

<210> 26
<211> 19
<212> DNA
<213> Artificial Sequence

<220>
<223> PCR/sequencing primer to verify mutant

<400> 26
ctcggtgacc accgaccac                                                    19

<210> 27
<211> 20
<212> DNA
<213> Artificial Sequence

<220>
<223> PCR primer to verify the presence of plasmid pSW-2

<400> 27
ggacgcttcg ctgaaaacta                                                   20

<210> 28
<211> 20
<212> DNA
<213> Artificial Sequence

<220>
<223> PCR primer to verify the presence of plasmid pSW-2

<400> 28
aacgtcgtga ctgggaaaac                                                   20

## Description

### FIELD OF THE INVENTION

The present invention is concerned with the production of compounds derived from oxaloacetate, such as ectoine or its biosynthetic precursor Ny-acetyl-L-2,4-diaminobutyric acid (NADA) or hydroxyectoine, in genetically modified microorganisms. The genetic modifications increase the obtainable yield by different mechanisms, such as by inhibiting the intracellular degradation of the compound, by increasing the extracellular accumulation of the compound, and by disrupting metabolic cycles, which are an obstacle for obtaining an increased yield. The invention is also concerned with the genetically modified microorganisms, and their use in the production of the desired compounds.

### BACKGROUND

Chemical compounds derived from oxaloacetate, such as ectoine or its precursor Nγ-acetyl-L-2,4-diaminobutyric acid (NADA), or hydroxyectoine, play an increasingly important role in several industries, for example as a cosmetics product and/or a medicinal product. Ectoine and hydroxyectoine have a lubricating and cell-protecting effect in creams and lotions, but can also protect the skin from harmful environmental influences such as UV radiation, allergies and fine dust. Furthermore, the precursor NADA is also a building block for polymers.

Alongside products in the cosmetics industry, ectoine is also a firm component in preparations which are used in the treatment of eye disorders. For example, ectoine in eye drops relieves irritation and inflammation by stabilizing the watery layer and the lipid layer of the tear film and therefore ensuring optimal lubrication.

In medicine, ectoine is used for irritation or inflammation of the skin or the mucous membranes, for example with a dry nose, allergic rhinitis (hay fever) or for inflammatory skin disorders such as neurodermatitis or psoriasis.

For use in the cosmetics industry, ectoine must have a purity of at least 80%. The degree of purity of ectoine for medicinal use is significantly higher. Currently, ectoine is an expensive and much sought-after product. Therefore there is a need for a method to provide ectoine, or its precursor NADA, in an efficient way and with high yields.

Ectoine (1,4,5,6-tetrahydro-2-methyl-4-pyrimidinecarboxylic acid) is a natural compound found in several species of bacteria. Ectoine is found in high concentrations in halophilic microorganisms and confers resistance towards salt and temperature stress. Ectoine was first identified in the microorganism *Ectothiorhodospira halochloris,* but has since been found in a wide range of Gram-negative and Gram-positive bacteria. Other species of bacteria in which ectoine was found include *Brevibacterium linens, Halomonas elongata*, *Marinococcus halophilus*, *Pseudomonas stutzeri*, *Halomonas titanicae*, *Halorhodospira halophila*, and *Halomonas ventosae.*

Ectoine is synthesized in three successive enzymatic reactions starting from aspartic β-semialdehyde. The enzyme ectoine synthase, encoded by the gene *ectC*, catalyzes the circularization of NADA to ectoine.

Ectoine is currently produced on an industrial scale by high-density fermentation of salt-tolerant bacteria such as *Halomonas elongata*, which in its wild-type status carries the genetic information to produce ectoine. Attempts have been made to increase the natural ectoine production by genetically modifying the microorganism.

It has been found that *Halomonas elongata* cells do not rely only on *de novo* synthesis of ectoine for adaptation to high saline environments, but can also take up compatible solutes or precursors thereof from the medium. To enable solute uptake, *H. elongata* is equipped with a set of compatible solute transporters of which only one accepts ectoine as a substrate, namely, the ectoine-specific transporter TeaABC. *H. elongata* with an inoperable TeaABC transporter constantly releases ectoine to the surrounding medium. Despite this, cells of this mutant are able to keep the internal ectoine concentration at the same level as the wild-type strain. Apparently, the mutation of teaABC not only causes excretion of ectoine to the medium but also results in overproduction of ectoine. An ectoine production strain of *H. elongata* with higher productivity in ectoine synthesis than the wild-type strain was designed (EP 1 409 707 B1). This mutant is also called "leaky mutant", as the uptake mechanism is compromised.

A further attempted approach to increase ectoine production was to prevent ectoine degradation in the cells producing ectoine. It was found that the deletion of gene *doeA*, encoding the ectoine hydrolase protein, which catalyzes the first step in the ectoine degradation pathway, can lead to increased volumetric productivity of ectoine (Schwibbert et al. 2011).

While these attempts managed to increase the yield obtainable from microorganisms producing ectoine, there is still a need for a method, which further increases this yield. Furthermore, there is a need for microorganisms, which are capable of producing ectoine at this high level to make the high yield obtainable.

The present invention provides such an improved method for producing ectoine or other chemical compounds derived from oxaloacetate metabolism in microorganisms by providing microorganisms capable of producing the compound at this high level. The microorganisms comprise a novel combination of genetic modifications responsible for the high yield.

### SUMMARY OF THE INVENTION

The present invention provides a method according to the appended claims.

The present invention provides a method for producing a chemical compound derived from oxaloacetate metabolism in microorganisms, wherein the microorganism comprises the genetic information for the production of the chemical compound, wherein the microorganism is genetically modified by
increasing the extracellular accumulation of the chemical compound by disruption of the uptake mechanism of the chemical compound or by introduction of efflux channels, or by reversible permeabilization of the cytoplasmic membrane, or by replacing passive channels with active transporters; and
disrupting the intracellular degradation mechanism of the chemical compound; and disrupting the PEP-pyruvate-oxaloacetate node to increase the production of the chemical compound.

In the method of the present invention the microorganism can be a gram negative bacterium, wherein optionally the bacterium can be a halophilic bacterium, wherein optionally the microorganism can be selected from the group comprising *Halomonas elongata*, *Brevibacterium linens, Ectothiorhodospira halochloris*, *Marinococcus halophilus*, *Pseudomonas stutzeri*, *Halomonas titanicae*, *Halorhodospira halophila*, and *Halomonas ventosae.*

The medium used to cultivate the mutated microorganisms of the present invention in the method of the present invention preferably comprises a sodium concentration of at least 0.5 M, preferably between to 0.5 M and 1 M.

The chemical compound derived from oxaloacetate metabolism can be ectoine or hydroxyectoine.

The chemical compound derived from oxaloacetate metabolism can be Nγ-acetyl-L-2,4-diaminobutyric acid (NADA).

The microorganism can further comprise a deletion of the ectoine synthase gene *ectC,* when the desired end product is NADA.

In the method of the present invention, the uptake mechanism of the chemical compound can be disrupted by disruption of a TRAP transporter, wherein preferably the TRAP transporter can be TeaABC. TeaABC can be disrupted by deletion of at least one of the genes selected from the group comprising *teaA*, *teaB*, and teaC, wherein optionally all of the genes in the group can be deleted.

In the method of the present invention, the degradation mechanism of the chemical compound can be disrupted by deletion of the ectoine hydrolase gene *doeA*.

In the method of the present invention, the PEP-pyruvate-oxaloacetate node can be disrupted by deletion of the phosphoenolpyruvate carboxykinase gene *Pck,* and/or the deletion of PEP carboxylase gene *Ppc,* and/or the deletion of the malic enzyme gene maeB, and or *maeA.* Optionally, the genes *Pck*, *Ppc*, *maeB,* and *maeA* are deleted.

The present invention also provides a microorganism comprising the genetic information for the production of a chemical compound derived from oxaloacetate metabolism, comprising the following genetic modifications:
increase of the extracellular accumulation of the chemical compound by disruption of the uptake mechanism of the chemical compound or by introduction of efflux channels, or by reversible permeabilization of the cytoplasmic membrane, or by replacing passive channels with active transporters; and
disruption of the intracellular degradation mechanism of the chemical compound; and
disruption of the PEP-pyruvate-oxaloacetate node to increase the production of the chemical compound.

In the microorganism of the present invention, the uptake mechanism of the chemical compound can be disrupted by disruption of a TRAP transporter, wherein preferably the TRAP transporter can be TeaABC. The degradation mechanism of the chemical compound can be disrupted by deletion of the ectoine hydrolase gene *doeA.* The PEP-pyruvate-oxaloacetate node can be disrupted by deletion of the phosphoenolpyruvate carboxykinase gene *Pck,* and/or the deletion of PEP carboxylase gene *Ppc,* and/or the deletion of the malic enzyme gene *mae*B, and or *maeA.*

The microorganism of the present invention can be a gram negative bacterium, wherein optionally the bacterium is a halophilic bacterium, or wherein the microorganism is selected from the group comprising *Halomonas elongata*, *Brevibacterium linens, Ectothiorhodospira halochloris*, *Marinococcus halophilus*, *Pseudomonas stutzeri*, *Halomonas titanicae*, *Halorhodospira halophila*, and *Halomonas ventosae.*

The present invention also provides the use of the microorganism of the invention for producing a chemical compound derived from oxaloacetate metabolism, such as ectoine, hydroxyectoine, or NADA.

### BRIEF DESCRIPTION OF THE DRAWINGS

**Figure 1** shows the PEP-Pyr-OAA node and the glyoxylate shunt in *H. elongata* as it has been determined by genomic analysis.
**Figure 2** shows ectoine concentrations in medium in overnight cultures of three strains of *Halomonas elongata* in mineral medium (MM63); each value is the average of three cultures. Error bars represent two standard deviations. The strains are WT, which is the "leaky mutant" (ΔTeaABCΔDoeA), KH1(ΔTeaABCΔDoeAΔPck) and KH2(ΔTeaABCΔDoeAΔPckΔMaeB). A measurement was removed as an outlier according to the interquartile range criterion (x> Q3+1.5*IQR).

### DESCRIPTION OF THE INVENTION

It has been surprisingly found that the obtainable yield of a chemical compound derived from oxaloacetate metabolism such as ectoine in microorganisms can be significantly increased by introducing at least one genetic modification which disrupts the PEP-pyruvate-oxaloacetate node into microorganisms already comprising genetic modifications to increase the extracellular accumulation of the chemical compound, for example by disrupting the uptake mechanism of the chemical compound, and disrupting the intracellular degradation mechanism of the chemical compound.

It has been found that the synthesis of ectoine from its precursor oxaloacetate (OAA) withdraws a high amount of carbon out of the tricarboxylic acid (TCA) cycle, which must then be replenished by anaplerotic reactions. The PEP-pyruvate-oxaloacetate node has been found to play a major role in these anaplerotic reactions.

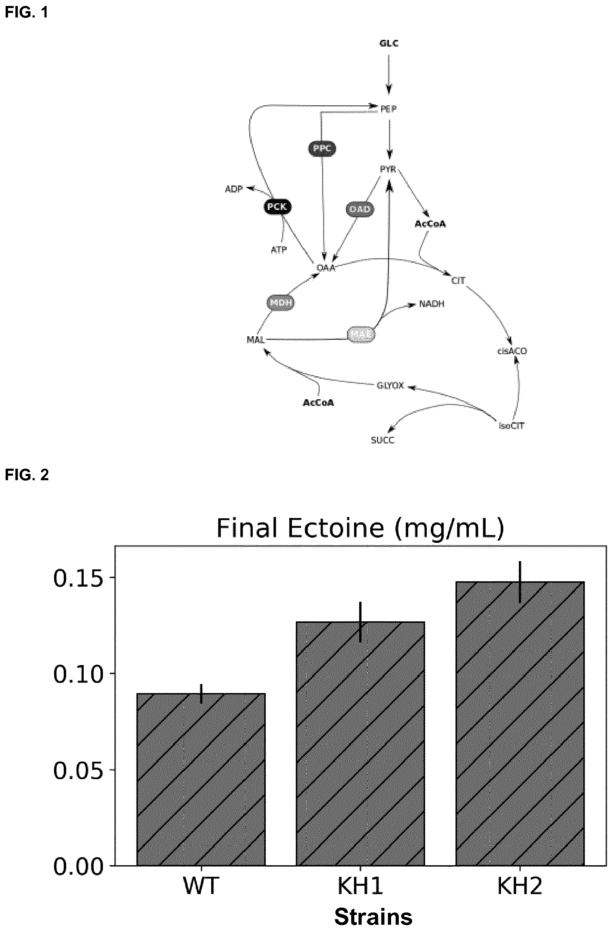

**Figure 1** shows the PEP-Pyr-OAA node and the glyoxylate shunt in *H. elongata* as it has been determined by genomic analysis. It has been shown that two of the four enzymes shown in **Figure 1** contribute to the anaplerotic flux: PEP carboxylase (Ppc) and oxaloacetate decarboxylase (Oad). The other two enzymes: Phosphoenolpyruvate carboxykinase (Pck) and malic enzyme (Mae) carry flux in the opposite direction when needed, be it for gluconeogenesis or, in the case of Mae, as part of a bypass to malic dehydrogenase (also shown in **Figure 1**).

It has now been surprisingly found that disrupting the PEP-Pyr-OAA node by deletion of Pck, Ppc and/or Mae, mutant strains can be obtained, which are capable of a further increased yield of chemical compounds derived from OAA. Mae can be present in two isoforms, MaeA and MaeB. Both are targets for deletion according to the present invention.

It has been found that these mutations lead to an improved ectoine production in two different ways.

The first effect of the mutations introduced, separately or simultaneously, is to increase the supply of OAA and to suppress futile cycles. A futile cycle, also known as a substrate cycle, occurs when two metabolic pathways run simultaneously in opposite directions and have no overall effect other than to dissipate energy in the form of heat. For example, if glycolysis and gluconeogenesis were to be active at the same time, glucose would be converted to pyruvate by glycolysis and then converted back to glucose by gluconeogenesis, with an overall consumption of ATP.

The increase in supply of OAA has been shown to disrupt the normal regulation of the PEP-Pyr-OAA node in other gram negative bacteria and would not be expected to lead to an increase of ectoine production by itself. Rather, the increase in supply could lead to a general decrease in metabolic activity due partly to the inhibition of Ppc, as observed in *E*. *coli.* It has however been unexpectedly found that this decrease in metabolic activity can be mitigated by combining two strategies.

The first strategy is to ensure that an alternative anaplerotic enzyme that is regulated by different effectors is present in the cell. In the particular case of *H. elongata* the wild type already possesses such an enzyme, Oad, which has been shown to be regulated by sodium concentration in the medium. In the presence of a significant salt concentration such as 0.5 M to 1 M a significant anaplerotic flux is thus guaranteed in *H. elongata*. The presence of the alternative anaplerotic enzyme can also be ensured by introduction of the genetic information for the enzyme into the cell by artificial means.

The second strategy is to increase the demand for the desired end product to at least partially compensate the effects of increased supply on the metabolic state of the cell. This can be done by several mechanisms, which result in a reduction of the concentration of the desired end product in the cell.

It can either be ensured that the desired end product is removed from the cell, and/or it can be ensured that desired end product already removed from the cell cannot be taken up again by the cell.

Removing the desired end product from the cell can for example be achieved by introduction of efflux channels into the cell, or by permeabilization of the membrane by chemical agents such as organic solvents, e.g. methanol, acetone, or detergents such as saponin, triton or tween, or by replacement of passive channels with active transporters. Furthermore, genes encoding proteins acting as active transporters could be overexpressed in a mutant strain to achieve increased removal of the desired end product from the cell.

To ensure that any desired end product already removed from the cell into the medium is not taken up again by the cell, the uptake mechanism can be disrupted, e.g. by deleting genes encoding the proteins which facilitate the uptake. In the case of *H. elongata*, the disruption of the uptake mechanism can be carried out by deletion of the genes encoding the TeaABC transporter. This has already been done in the art and the resulting mutant *H*. *elongata* strain has been termed "leaky mutant" (ΔTeaABCΔDoeA).

The second effect of the mutations introduced, separately or simultaneously, is to optimize the process by decoupling growth and ectoine production. Independently of the effect of increased OAA supply, the deletion of the genes *Pck*, *Ppc*, *maeB* and/or *maeA* eliminates the two reactions that are important for gluconeogenesis. This will hinder growth on several carbon sources such as malate and acetate, since any decarboxylation of TCA intermediates would have to proceed through reactions that are thermodynamically unfavorable. Thus, mutant strains according to the invention can use glucose for both growth and ectoine production, but gluconeogenic carbon sources will only be usable for ectoine production. It has been shown by the applicants of the present invention that a microorganism which is genetically modified as defined above allows an increase of the ectoine yield of about at least 50 % (see also **Figure 2**).

The present invention is therefore concerned with a method for producing a chemical compound derived from oxaloacetate metabolism in microorganisms, wherein the microorganism comprises the genetic information for the production of the chemical compound, wherein the microorganism is genetically modified by
- increasing the extracellular accumulation of the chemical compound by disruption of the uptake mechanism of the chemical compound or by introduction of efflux channels, or by reversible permeabilization of the cytoplasmic membrane, or by replacing passive channels with active transporters; and
- disruption of the intracellular degradation mechanism of the chemical compound; and
- disruption of the PEP-pyruvate-oxaloacetate node to increase the production of the chemical compound.

Disruption of the PEP-pyruvate-oxaloacetate node to increase the production of the chemical compound can be achieved by silencing the function of enzymes involved in the PEP-pyruvate-oxaloacetate node. Preferred enzymes are Ppc, Pck, MaeA, and MaeB. Their function can for example be silenced by deletion of the sequence comprising the genetic information for the enzyme.

The PEP-pyruvate-oxaloacetate node can be disrupted by deletion of the phosphoenolpyruvate carboxykinase gene *Pck,* and/or by deletion of the PEP carboxylase gene *Ppc* and/or the deletion of the malic enzyme gene *maeB*, and/or *maeA.*

In a preferred embodiment the *Pck* gene and the *maeB* gene are both deleted.

In a further preferred embodiment the *Pck* gene, the *Ppc* gene and the *maeB* gene are deleted. In a yet further preferred embodiment the *Pck* gene, the *Ppc* gene, the *maeB* gene, and the maeA gene are deleted.

The chemical compound derived from oxaloacetate metabolism can be ectoine or hydroxyectoine. The chemical compound derived from oxaloacetate metabolism can also be Nγ-acetyl-L-2,4-diaminobutyric acid (NADA). In this embodiment, the microorganism can further comprise a deletion of the ectoine synthase gene *ectC* to allow accumulation of NADA. The chemical compound derived from oxaloacetate metabolism can also be one of a whole family of amino acids such as aspartate, asparagine, methionine, lysine and threonine. The person of skill in the art will know how to disrupt the degradation mechanism of these compounds, and the person of skill in the art will also know how to increase extracellular accumulation of these compounds, and will therefore be able to adapt the teachings of this application to a method directed at increasing the yield of chemical compounds derived from oxaloacetate metabolism other than NADA, ectoine, or hydroxyectoine.

The microorganism can be a gram negative bacterium, wherein optionally the bacterium is a halophilic bacterium. The microorganism can be selected from the group comprising *Halomonas elongata*, *Brevibacterium linens*, *Ectothiorhodospira halochloris*, *Marinococcus halophilus*, *Pseudomonas stutzeri*, *Halomonas titanicae*, *Halorhodospira halophila*, and *Halomonas ventosae.*

The uptake mechanism of the chemical compound can be disrupted by disruption of a TRAP transporter, wherein preferably the TRAP transporter is TeaABC. TeaABC can be disrupted by deletion of at least one of the genes selected from the group comprising *teaA*, *teaB*, and *teaC*, wherein optionally all of the genes in the group are deleted. Based on the information provided in this application the person of skill in the art will be able to identify similar targets in other microorganisms, which have the same function as the TRAP transporter TeaABC in *H. elongata.*

Disruption of the intracellular degradation of the compound can be achieved by several mechanisms known to the person of skill in the art. For example, genes in the degradation pathway can be knocked out or knocked down. The translation of these proteins can also be inhibited or silenced by antisense or interference RNA targeting the mRNA of the genes encoding the proteins involved in the degradation pathway. The gene expression can also be downregulated for example by promoter replacement or by using the CRISPR/CAS technique. In the case of *H. elongata* disruption of the intracellular degradation of ectoine has already been achieved by deletion of the ectoine hydrolase gene *doeA,* which catalyzes the first step in the ectoine degradation pathway. Based on the information provided in this application the person of skill in the art will be able to identify similar targets in other microorganisms, which have the same function as the *doeA* in *H. elongata.*

The medium used to cultivate the mutated microorganisms of the present invention preferably comprises a significant sodium concentration, such as 0.5 M to 1 M. Sea water has a sodium concentration of about 0.5 M and can therefore be used as a base medium to grow the mutants of the present invention. *E. coli* exhibits reduced growth at a sodium concentration of about 0.5 M and cannot grow when the sodium concentration exceeds 0.9 M. The significant sodium concentration ensures that the anaplerotic flux is maintained even when OAA supply is increased and therefore further improves the method of the present invention.

The present invention is also concerned with a microorganism obtained by the genetic modifications as defined above.

The present invention is also concerned with the use of the genetically modified microorganism for producing a chemical compound derived from oxaloacetate metabolism.

### EXAMPLES

### Example 1

### Strains, plasmids, and growth conditions

*Halomonas elongata* DSM2581T was routinely grown in minimal medium MM63 or LB medium containing 1 M NaCl at 30 °C under shaking. All *Escherichia coli* strains were grown in LB medium at 37 °C amended with the antibiotic necessary for maintenance of the plasmid. All plasmids and genetic manipulations were performed on *E. coli* DH5α or *E*. *coli* DH5α λ-pir.

### Example 2

### Construction of plasmids pSEVA212S-pckA::Sm, pSEVA212S-ΔpckA, pSEVA212S-maeB::Sm, pSEVA212S-ΔmaeB, and pSEVA212S-ΔmaeA

Replacement of the genes *pckA*, *maeB*, and *maeA* with *aadA*, encoding a Streptomycin resistance cassette was performed by adapting the method previously reported for *Pseudomonas putida* (Martínez-García *et al*., 2011). The plasmids were constructed by Gibson Assembly (NEB) according to the suppliers manual. Oligonucleotides used for the gene amplification are shown in **Table 1**. A DNA segment containing about 500 bp of the genomic sequence upstream of the target gene (*pckA*, *maeB*, or *maeA*) followed by the sequence of the *aadA* gene, encoding the streptomycin resistance cassette, and about 500 bp of the downstream chromosomal region, was inserted into pSEVA212S, carrying the R6K suicide origin of replication (Martínez-García *et al*., 2015) previously linearized with EcoRI. The plasmid was transformed into *E. coli* DH5α λ-pir and clones carrying it were selected on LB agar plates supplemented with 200 µg/ml streptomycin (Sm 200). The correct assembly of pSEVA212S-pckA::Sm, pSEVA212S-ΔpckA, pSEVA212S-maeB::Sm, pSEVA212S-ΔmaeB, and pSEVA212S-ΔmaeA was verified by isolation of the plasmids and sequencing.

### Example 3

### Conjugal transfer of plasmids into H. elongata by triparental mating

Two plasmids had to be transferred to *H. elongata* using *E. coli* HBH101 (pRK600) as helper in the mating procedure. First, plasmid pSW-2 encoding the 1-Scel endonuclease was transferred to *H. elongata*. Therefore, the donor strain *E. coli* DH5α λ-pir (pSW-2), the recipient strain *H. elongata*, and the helper strain *E. coli* HBH101 (pRK600) were grown in LB medium, amended with the antibiotic necessary to assure plasmid maintenance (pSW-2: 10 µg/ml gentamycin and pRK600: 35 µg/ml chloramphenicol) to the stationary phase (overnight). The next day, optical densities at 600 nm were measured and 1 ml of the donor culture was mixed with the amount of recipient and helper culture to obtain OD ratios of 1:1:1 and 1:2:2. These suspensions were centrifuged; the supernatant was discarded by decanting and the cell pellet was resuspended in the remaining medium rest. This condensed cell suspension was pipetted on one spot of a LB agar plate containing 0.5 M NaCl, which was incubated for 5 h at 30 °C. Afterwards, the cells were resuspended in 600 µl of a 0.5 M NaCl solution, an aliquot of 100 µl was plated on LB agar plates containing 0.5 M NaCl, 500 µg/ml ampicillin and 50 µg/ml gentamycin and the plates were incubated for 48 h at 30 °C. Colonies were picked and the presence of pSW-2 was verified by PCR with pSW2-F and pSW2-R (see Table 1).

Next, pSEVA212S-pckA::Sm, pSEVA212S-ΔpckA, pSEVA212S-maeB::Sm, pSEVA212S-ΔmaeB, and pSEVA212S-ΔmaeA had to be transferred to *H. elongata* (pSW-2) again by triparental mating. This was performed as described above but using *H*. *elongata* (pSW-2) as recipient, *E. coli* DH5α λ-pir (pSEVA212S-pckA::Sm, pSEVA212S-ΔpckA, pSEVA212S-maeB::Sm, pSEVA212S-ΔmaeB, and pSEVA212S-ΔmaeA) as donor and *E. coli* HBH101 (pRK600) as helper. The cointegrates were selected by plating different dilutions of the mating mixture on LB agar plates containing 0.5 M NaCl, 500 µg/ml ampicillin, 60 µg/ml kanamycin, and 50 µg/ml gentamycin.

### Example 4

### Resolution of cointegrates to obtain H. elongata ΔpckA::Sm, ΔMaeB::Sm and ΔMaeA::Sm

To resolve the cointegrates a single colony was picked from the selective agar plate and grown in 3 ml LB medium with 1 M NaCl, 500 µg/ml ampicillin, 200 µg/ml streptomycin and 50µg/ml gentamycin overnight at 30 °C. The next day, cells were pelleted by centrifugation, washed once in LB medium containing 1 M NaCl, and subsequently resuspended to an OD600 of 1 with LB medium with 1 M NaCl, 500 µg/ml ampicillin, and 50µg/ml gentamycin. Four ml of that culture were induced with 80 µl of 3-methylbenzoate to a final concentration of 10 mM and incubated at 30 °C under shaking.

After 3.5 h of induction of 1-Scel expression an aliquot was plated on LB agar with 1 M NaCl, 500 µg/ml ampicillin and 200 µg/ml streptomycin and incubated at 30 °C for 48 hours. Single colonies were picked and gridded on LB plates with 1 M NaCl, 500 µg/ml ampicillin, and either 60 µg/ml kanamycin or 200 µg/ml of streptomycin to identify those clones, that lost the kanamycin resistance cassette but maintained the streptomycin cassette. Clones showing the desired phenotype should represent the desired mutants. This was verified by PCR using the primers as shown in Table 1 that hybridized to sequences outside the 500 bp flanks used in pSEVA ΔpckA::Sm, ΔMaeB::Sm or ΔMaeA::Sm and by sequencing of the whole fragment.

**Table 1: Oligonucleotides used in this work.**

| **SEQ ID NO:** | **Name** | **Sequence** | **Function** |
|---|---|---|---|
| SEQ ID NO:1 | pckA_up_fwdP | atccccgggtaccgagctcgGATGTCTGGAAGATTACTTCAGC | construction of plasmid pSEVA212S-*pckA*::Sm |
| SEQ ID NO:2 | pckA_up_revP | gtcaaggttcGTCGATTCCTGGGCCTTG | construction of plasmid pSEVA212S-*pckA*::Sm |
| SEQ ID NO:3 | pckA_Strep_fwdP | aggaatcgacGAACCTTGACCGAACGCAG | construction of plasmid pSEVA212S-*pckA*::Sm |
| SEQ ID NO:4 | pckA_Strep_revP | ctcggcaggcTTATTTGCCGACTACCTTGGTG | construction of plasmid pSEVA212S-*pckA*::Sm |
| SEQ ID NO:5 | pckA_down_fwdP | cggcaaataaGCCTGCCGAGGGCGACAAC | construction of plasmid pSEVA212S-*pckA*::Sm |
| SEQ ID NO:6 | pckA_down_revP | agggataacagggtaatctgCAGGTCCAGATGATCGCGACAAGG | construction of plasmid pSEVA212S-*pckA*::Sm |
| SEQ ID NO:7 | pckA_up_revPII | ctcggcaggcGTCGATTCCTGGGCCTTG | construction of plasmid pSEVA212S-Δ*pckA* |
| SEQ ID NO:8 | pckA_down_fwdPII | aggaatcgacGCCTGCCGAGGGCGACAAC | construction of plasmid pSEVA212S-Δ*pckA* |
| SEQ ID NO:9 | SeqP_DelpckA_fwdP | GAAGGGCAGATCACCATCAC | PCR/sequencing primer to verify mutant |
| SEQ ID NO:10 | SeqP_DelpckA_revP | GTCGAGCAGTTCCACGGTAG | PCR/sequencing primer to verify mutant |
| SEQ ID NO:11 | M_pSEVA212_F | atccccgggtaccgagctcgTCCGAAGGGTACGCGTACCC | construction of plasmid pSEVA212S-*maeB*::Sm |
| SEQ ID NO:12 | M_up/sm_R | gtcaaggttcGCTGAGGATCCGATCTGCGATTC | construction of plasmid pSEVA212S-*maeB*::Sm |
| SEQ ID NO:13 | M_up/sm_F | gatcctcagcGAACCTTGACCGAACGCAGC | construction of plasmid pSEVA212S-*maeB*::Sm |
| SEQ ID NO:14 | M_sm/down_R | ttaagcagtaTTATTTGCCGACTACCTTGGTG | construction of plasmid pSEVA212S-*maeB*::Sm |
| SEQ ID NO:15 | M_sm/down_F | cggcaaataaTACTGCTTAAATGCCTGCACGTGC | construction of plasmid pSEVA212S-*maeB*::Sm |
| SEQ ID NO:16 | M_pSEVA212_R | agggataacagggtaatctgCTGCGCGGCGAGATACGTCA | construction of plasmid pSEVA212S-*maeB*::Sm |
| SEQ ID NO:17 | M_up/down_F | ttaagcagtaGCTGAGGATCCGATCTGCGATTC | construction of plasmid pSEVA212S-Δ*maeB* |
| SEQ ID NO:18 | M_up/down_R | gatcctcagcTACTGCTTAAATGCCTGCACGTG | construction of plasmid pSEVA212S-Δ*maeB* |
| SEQ ID NO:19 | gHe_M_F | AACACCGACAGCCAGACATT | PCR/sequencing primer to verify mutant |
| SEQ ID NO:20 | gHe_M_R | TTGGACAGCGGGAGTTCG | PCR/sequencing primer to verify mutant |
| SEQ ID NO:21 | MA_EcoRI_up_F | ttttgaattcGCAACCACGAGATCATG | construction of plasmid pSEVA212S-Δ*maeA* |
| SEQ ID NO:22 | MA_up_R | agacgaaccgGGAAACACTGCTTGAATGTCTG | construction of plasmid pSEVA212S-Δ*maeA* |
| SEQ ID NO:23 | MA_down_F | cagtgtttccCGGTTCGTCTCTACTGTCTC | construction of plasmid pSEVA212S-Δ*maeA* |
| SEQ ID NO:24 | MA_HindIII_down_R | aaaaaagcttCTGGATGCTCTCCTGCGT | construction of plasmid pSEVA212S-Δ*maeA* |
| SEQ ID NO:25 | gHe_MA_F | CAAGCGGGTCCAGCAGTC | PCR/sequencing primer to verify mutant |
| SEQ ID NO:26 | gHe_MA_R | CTCGGTGACCACCGACCAC | PCR/sequencing primer to verify mutant |
| SEQ ID NO:27 | pSW2 F | GGACGCTTCGCTGAAAACTA | PCR primer to verify the presence of plasmid pSW-2 |
| SEQ ID NO:28 | pSW2 R | AACGTCGTGACTGGGAAAAC | PCR primer to verify the presence of plasmid pSW-2 |

### Example 5

### Ectoine production by H. elongata mutants of the present invention compared to ectoine production by an H. elongata mutant of the prior art

The result of this experiment is shown in **Figure 2****.**

**Figure 2** shows ectoine concentrations in medium in overnight cultures of three strains of *Halomonas elongata* in mineral medium (MM63) each value is the average of three cultures. Error bars represent two standard deviations. The strains are WT, the "leaky mutant" (ΔTeaABCΔDoeA) of the prior art, and KH1(ΔTeaABCΔDoeAΔPck) and KH2(ΔTeaABCΔDoeAΔPckΔMaeB) of the present invention. A measurement was removed as an outlier according to the interquartile range criterion (x> Q3+1.5*IQR).

It is shown that the mutants of the present invention exhibit a significantly increased yield in ectoine production compared to the mutant of the prior art.

### REFERENCES

- EP1409707B1
- Schwibbert K. et al. A blueprint of ectoine metabolism from the genome of the industrial producer Halomonas elongata dsm 2581t. Environmental microbiology, 13(8):1973-1994,2011.
- Kindzierksi V. et al. Osmoregulationin the halophilic bacterium Halomonas elongata: A case study for integrative systems biology. PLOS ONE, 12(1):e0168818, 2017
- Esteban Martínez-García and Victor de Lorenzo. Engineering multiple genomic deletions in gram-negative bacteria: analysis of the multiresistant antibiotic profile of Pseudomonas putida kt2440. Environmental microbiology, 13(10):2702-2716, 2011.
- Esteban Martínez-García, Tomás Aparicio, Angel Goñi-Moreno, Sofia Fraile, and Victor de Lorenzo. Seva 2.0: an update of the standard european vector architecture for de-/re-construction of bacterial functionalities. Nucleic acids research, 43(D1):D1183-D1189,2015

## Claims

1. Method for producing a chemical compound derived from oxaloacetate metabolism in a microorganism, wherein the microorganism comprises the genetic information for the production of the chemical compound, wherein the microorganism is genetically modified by
a) increasing the extracellular accumulation of the chemical compound by disruption of the uptake mechanism of the chemical compound or by introduction of efflux channels, or by reversible permeabilization of the cytoplasmic membrane, or by replacing passive channels with active transporters; and
b) disrupting the intracellular degradation mechanism of the chemical compound; and
c) disrupting the PEP-pyruvate-oxaloacetate node to increase the production of the chemical compound.

2. The method of claim 1, wherein the microorganism is a gram negative bacterium, wherein optionally the bacterium is a halophilic bacterium, wherein optionally the microorganism is selected from the group comprising *Halomonas elongata*, *Brevibacterium linens, Ectothiorhodospira halochloris*, *Marinococcus halophilus*, *Pseudomonas stutzeri*, *Halomonas titanicae*, *Halorhodospira halophila*, and *Halomonas ventosae.*

3. The method of claim 1 or 2, wherein a medium used to cultivate the mutated microorganism of the present invention preferably comprises a sodium concentration of at least 0.5 M, preferably between 0.5 M and 1 M.

4. The method of any one of the preceding claims, wherein the chemical compound derived from oxaloacetate metabolism is ectoine or hydroxyectoine.

5. The method of any one of the preceding claims, wherein the chemical compound derived from oxaloacetate metabolism is Nγ-acetyl-L-2,4-diaminobutyric acid (NADA).

6. The method of claim 5, wherein the microorganism further comprises a deletion of the ectoine synthase gene *ectC.*

7. The method of any one of the preceding claims, wherein the uptake mechanism of the chemical compound is disrupted by disruption of a TRAP transporter, wherein preferably the TRAP transporter is TeaABC.

8. The method of claim 7, where TeaABC is disrupted by deletion of at least one of the genes selected from the group comprising *teaA*, *teaB*, and *teaC*, wherein optionally all of the genes in the group are deleted.

9. The method of any one of the preceding claims, wherein the degradation mechanism of the chemical compound is disrupted by deletion of the ectoine hydrolase gene *doeA.*

10. The method of any one of the preceding claims, wherein the PEP-pyruvate-oxaloacetate node is disrupted by deletion of the phosphoenolpyruvate carboxykinase gene *Pck,* and/or the deletion of PEP carboxylase gene *Ppc,* and/or the deletion of the malic enzyme gene *mae*B, and or *maeA.*

11. Microorganism comprising the genetic information for the production of a chemical compound derived from oxaloacetate metabolism, comprising the following genetic modifications:
a) increase of the extracellular accumulation of the chemical compound by disruption of the uptake mechanism of the chemical compound or by introduction of efflux channels, or by reversible permeabilization of the cytoplasmic membrane, or by replacing passive channels with active transporters; and
b) disruption of the intracellular degradation mechanism of the chemical compound; and
c) disruption of the PEP-pyruvate-oxaloacetate node to increase the production of the chemical compound.

12. The microorganism of claim 11, wherein the uptake mechanism of the chemical compound is disrupted by disruption of a TRAP transporter, wherein preferably the TRAP transporter is TeaABC, and wherein the degradation mechanism of the chemical compound is disrupted by deletion of the ectoine hydrolase gene *doeA,* and wherein PEP-pyruvate-oxaloacetate node is disrupted by deletion of the phosphoenolpyruvate carboxykinase gene *Pck,* and/or the deletion of PEP carboxylase gene *Ppc,* and/or the deletion of the malic enzyme gene *maeB*, and or *maeA.*

13. The microorganism of claim 11 or 12, wherein the microorganism is a gram negative bacterium, wherein optionally the bacterium is a halophilic bacterium, or wherein the microorganism is selected from the group comprising *Halomonas elongata*, *Brevibacterium linens, Ectothiorhodospira halochloris, Marinococcus halophilus*, *Pseudomonas stutzeri*, *Halomonas titanicae*, *Halorhodospira halophila*, and *Halomonas ventosae.*

14. Use of the microorganism of any one of claims 11 to 13 for producing a chemical compound derived from oxaloacetate metabolism according to the method of any one of claims 1 to 10.

15. Use of claim 14, wherein the chemical compound is ectoine, hydroxyectoine, or NADA.
